Europäisches Patentamt

European Patent Office

Office européen des brevets

(19)

(11) Publication number: **0 391 621**
**A1**

## (12) EUROPEAN PATENT APPLICATION

(21) Application number: 90303430.4

(22) Date of filing: 30.03.90

(51) Int. Cl.⁵: **C08B 31/12, A61K 49/04**

(30) Priority: 03.04.89 JP 81577/89

(43) Date of publication of application:
**10.10.90 Bulletin 90/41**

(84) Designated Contracting States:
**DE FR GB IT**

(71) Applicant: **Nippon Universal Pharmaceutical Co., Ltd.,**
**Shinjuku-Sumitomo Building 6-1, 2-chome**
**Nishishinjuku Shinjuku-ku**
**Tokyo(JP)**

(72) Inventor: **Suami, Tetsuo**
**5-8, Nakamachi 3-chome**
**Musashino-shi, Tokyo(JP)**
Inventor: **Sako, Masao**
**1-2, Nishiochiai 5-chome**
**Suma-ku, Kobe-shi, Hyogo-ken(JP)**
Inventor: **Kanda, Yoshihiro**
**1863-1-415, Hane, Hamuramachi**
**Nishitama-gun, Tokyo(JP)**

(74) Representative: **Ruffles, Graham Keith et al**
**MARKS & CLERK 57-60 Lincoln's Inn Fields**
**London WC2A 3LS(GB)**

(54) **Iodized hydroxyethyl starch of low viscosity and process for preparing the same.**

(57) An iodized hydroxyethyl starch of low viscosity having a limiting viscosity number of 0.03-0.13 and an iodine content of 13.5-25.0%. The iodized hydroxyethyl starch is useful as a contrast agent for X-ray examination, especially for opacifying vascular and lymphatic vessels. The hydroxyethyl starch having a high iodine content is readily soluble in water.

EP 0 391 621 A1

## Iodized hydroxyethyl starch of low viscosity and process for preparing the same

Background of the Invention

1. Field of the Invention

This invention relates to iodized hydroxyethyl starch of low viscosity, a process for preparing the same and a contrast agent for X-ray examination comprising said iodized hydroxyethyl starch of low viscosity.

The iodized hydroxyethyl starch of low viscosity of the invention is a novel compound which has not been disclosed in any literature. It is useful as a contrast agent for X-ray examination, especially for opacifying vascular and lymphatic vessels.

2. Description of the Prior Art

As instruments for the radiographic diagnosis were diffused and diagnostic methods including computer tomography (CT) were developed in recent years, use of contrast agents for diagnosis has been much increased in therapeutic practice.

Contrast agents for the vascular vessels currently widely used are those which comprise an iodine compound of benzoic acid as an opacifying component. Whereas these contrast agents are highly capable of opacifying the vessels under X-ray examination, they cause pain or feeling of heat in patients on introduction into the blood vessel. In addition, they are apt to cause allergic reactions of various degrees, and occasionally, death due to severe anaphylactic reactions are reported.

On the other hand, the lymphatic vessel contrast agent primarily used is an ethyl ester of poppy oil-fatty acids. The contrast agent, which comprises oily iodine compounds as the opacifying component, is not leaked through the wall of the lymphatic vessels, reaches the desired site and allows the vessels to cast distinct densities. However, since composition of the agent is oily, it may cause peripheral embolism when passed from the lymphatic system to the vascular system. It is known that after opacifying in the lymphatic system, there are caused frequent pulmonary embolism as well as embolism in the arterial system with severe adverse reactions such as cerebral infarct.

In order to solve these problems, we already proposed 6-iodoethylated starch (Japanese Patent Application Laid-Open-to-Public No. 272201/1986). As the 6-iodoethylated starch is a water-soluble macromolecular compound, it is superior to conventional contrast agents in residence in the blood vessels and is a safe compound without fear of occurrence of peripheral embolism. However, as the compound has an iodine content of as low as 12.5% and a higher limiting viscosity number of 0.19, the opacifying ability under X-ray examination is not sufficient and rapid introduction into blood vessels is difficult.

Summary of the Invention

As a result of extensive investigation to solve these problems, we have found that iodization of a hydroxyethylated starch having a limiting viscosity number of 0.04-0.18 produces iodized hydroxyethyl starch of low viscosity which has a high iodine content and is readily soluble in water. The invention has been completed on the basis of the above finding. It is unexpected that a high solubility in water is exerted despite increased iodine content as a hydrophobic group.

According to the present invention there are provided iodized hydroxyethyl starch of low viscosity characterized by having a limiting viscosity number of 0.03-0.13, preferably 0.05-0.07 and an iodine content of 13.5-25.0%, preferably 14.0-24.9% and contrast agents for X-ray examination comprising the same.

Further according to the invention there is provided a process for preparing said iodized hydroxyethyl starch of low viscosity which comprises reacting a hydroxyethylated starch having a limiting viscosity number of 0.04-0.18 with N-iodosuccinimide in the presence of triphenylphosphine.

The "limiting viscosity number" as referred to herein refers to the viscosity measured at 25°C by the Ubbelohde method.

The hydroxyethylated starch having a limiting viscosity number of 0.04-0.18 used in preparing the compounds of the invention is produced, for example, by etherifying a waxy starch mainly composed of amylopectin with ethylene oxide and subsequently acid hydrolyzing to a limiting viscosity number in a range between 0.04 and 0.18 (Japanese Patent Publication No. 39833/1970 and Japanese Patent Application Laid-Open-to-Public No. 69139/1979).

The iodized hydroxyethyl starch of low viscosity of the invention is prepared by reacting the above low viscosity hydroxyethylated starch with N-iodosuccinimide in the presence of triphenylphosphine.

The reaction is desirably carried out using an organic solvent such as dimethylformamide. The reaction temperature and the reaction time are about from room temperature to 100° C and for several minutes to 50 hours, although they are variable depending upon concentration of the reactants. Proportions of the reactants N-iodosuccinimide and triphenylphosphine used are suitably 1.0-1.7 moles per mole of the hydroxyethyl group in a starting starch. In order to increase iodine content in the product, the reagents are used in a proportion of 1.2 moles or more per mole of the hydroxyethyl group in the starting material, and a higher reaction temperature and a longer reaction time are applied. In order to produce a product having a higher limiting viscosity number or a lower degree of the molecular weight distribution, a lower reaction temperature and a shorter reaction time are applied, and a lager amount of a solvent is used. After completion of the reaction the desired product is collected from the reaction mixture in a conventional manner. For example, an alcohol such as methanol is added to the reaction mixture to decompose the excess of the reagents, and an appropriate organic solvent such as chloroform is added to the resulting mass followed by extraction with water. The extract is deionized by means of an ion-exchange resin, if needed, and concentrated under reduced pressure. To the concentrate is added an organic solvent such as acetone, methanol, ethanol or propanol, and the desired product is obtained by collecting precipitates thus formed. The product obtained can further be purified by means of an ultrafiltration or a column chromatography using Cephadex G25 or G50 (manufactured by Pharmacia) or Cephacryl S-200 (manufactured by Pharmacia).

It is believed that decomposition reactions may occur in part during the above-described iodization reaction to form an iodized hydroxyethyl starch of low viscosity of the invention having a lower molecular weight and reduced viscosity. Degree of the decomposition may be variable depending upon concentration of the reactants, reaction temperature and reaction time.

The iodized hydroxyethyl starch of low viscosity of the invention thus produced has the following physicochemical properties:

m.p.: 180-210° C (dec.)

IR: $\nu_{max}^{KBr}$

3400, 3380, 2930, 2920, 1640, 1630, 1625, 1360, 1350, 1015, 1010
Dissolution rate in water: 1-5 min.
Limiting viscosity number: 0.03-0.08
Iodine content: 13.5-25.0%

The iodized hydroxyethyl starch has a higher opacifying ability as the iodine content increases. However, if the content exceeds 25%, the compound will hardly be soluble in water. Also, whereas the higher is the viscosity, the longer is the residence time in blood vessels, introduction into blood vessel will be troublesome if the viscosity is too high.

The iodized hydroxyethyl starch of low viscosity of the invention is dissolved in distilled water for injection in a proportion of about 30-60% (w/v), and the solution is introduced into blood vessels or lymphatic vessel as a contrast agent for X-ray examination.

The compound of the invention has an $LD_{50}$ of 10,000 mg/kg or higher. No death is observed following consecutive daily administration of 5,000 mg/kg for 14 days.

The invention will be described in more detail with reference to Examples and Test Examples.

Example 1

In 40 ml of dry dimethylformamide was suspended with enough stirring 5.00 g of a low viscosity hydroxyethylated starch ($\eta$ 0.10) which had been dried under reduced pressure (1 mmHg) over phosphorus pentoxide at 100° C for 8 hours. To the suspension was added 2.60 g of triphenylphosphine which corresponded to a proportion of 1.0 mole per mole of the hydroxyethyl group in the starting material, and the mixture was stirred at room temperature for 40 min. to give a solution. Separately, 2.24 g of N-iodosuccinimide which corresponded to a proportion of 1.0 mole per mole of the same group was dissolved under cooling in 20 ml of dry dimethylformamide. The solution was added dropwise with stirring to the former solution of hydroxyethylated starch and triphenylphosphine under ice cooling over 18 min. The resulting brown-colored solution was heated with stirring in an oil bath at 70° C for 18 hours. After

3

completion of the reaction 10 ml of absolute methanol was added to the reaction mixture, which was then stirred at room temperature for 2 hours. The solvent was distilled off under reduced pressure while maintaining the reaction mixture at a temperature of 50°C or below to a concentrated volume of about 5-6 ml. To the residue was added 100 ml of chloroform, and the solution was extracted with 100 ml of distilled water. The aqueous layer was washed three times with 100 ml of chloroform and then passed through ion-exchange resin columns of 25 ml of Amberlite IR120B (manufactured by Organo, H$^+$ type) and 25 ml of IRA 400 (manufactured by Organo, OH$^-$ type), successively for deionization. The deionized solution was concentrated under reduced pressure at or below 40°C to about 10 ml. To the residual solution was added 60 ml of an acetone-isopropanol (1:1) solution, and a glutinous substance thus precipitated was isolated by decantation, to which was again added 60 ml of an acetone-isopropanol (1:1) solution to form powders. The powders were separated by filtration and dried. There was obtained 5.03 g of colorless powders.

m.p.: 202-206°C (dec.)

IR: $\nu_{max}^{KBr}$ cm$^{-1}$

3380, 2920, 1630, 1350, 1010

Elementary analysis

Found %: C, 39.23; H, 6.31; I, 16.74

Limiting viscosity number: 0.05

Example 2

In 48 ml of dry dimethylformamide was suspended with enough stirring 5.01 g of a low viscosity hydroxyethylated starch (n 0.10) which had been dried under reduced pressure (1 mmHg) over phosphorus pentoxide at 100°C for 8 hours. To the suspension was added 2.70 g of triphenylphosphine which corresponded to a proportion of 1.2 moles per mole of the hydroxyethyl group in the starting material, and the mixture was stirred at room temperature for 3 hours to give a solution. Separately, 3.13 g of N-iodosuccinimide which corresponded to a proportion of 1.2 moles per mole of the same group was dissolved under cooling in 24 ml of dry dimethylformamide. The solution was added dropwise with stirring to the former solution of hydroxyethylated starch and triphenylphosphine under ice cooling over 13 min. The resulting brown-colored solution was heated with stirring in an oil bath at 70°C for 18 hours. After completion of the reaction 10 ml of absolute methanol was added to the reaction mixture, which was then stirred at room temperature for 2 hours. The solvent was distilled off under reduced pressure while maintaining the reaction mixture at a temperature of 50°C or below to a concentrated volume of about 5-6 ml. To the residue was added 100 ml of chloroform, and the solution was extracted with 100 ml of distilled water. The aqueous layer was washed three times with 100 ml of chloroform and then passed through ion-exchange resin columns of 25 ml of Amberlite IR120B (H$^+$ type) and 25 ml of IRA 400 (OH$^-$ type), successively for deionization. The deionized solution was concentrated under reduced pressure at or below 40°C to about 10 ml. To the residual solution was added 60 ml of an acetone-isopropanol (1:1) solution, and a glutinous substance thus precipitated was isolated by decantation, to which was again added 60 ml of an acetone-isopropanol (1:1) solution to form powders. The powders were separated by filtration and dried. There was obtained 5.22 g of colorless powders. m.p.: 184-186°C (dec.)

IR: $\nu_{max}^{KBr}$ cm$^{-1}$

3400, 2930, 1640, 1360, 1015

Elementary analysis

Found %: C, 37.18; H, 6.16; I, 22.88

Limiting viscosity number: 0.05

Example 3

In 50 ml of dry dimethylformamide was suspended with enough stirring 5.00 g of a low viscosity hydroxyethylated starch ($\eta$ 0.10) which had been dried under reduced pressure (1 mmHg) over phosphorus pentoxide at 100°C for 8 hours. To the suspension was added 3.91 g of triphenylphosphine which corresponded to a proportion of 1.5 moles per mole of the hydroxyethyl group in the starting material, and the mixture was stirred at room temperature for 3 hours to give a solution. Separately, 3.36 g of N-iodosuccinimide which corresponded to a proportion of 1.5 moles per mole of the same group was dissolved under cooling in 20 ml of dry dimethyformamide. The solution was added dropwise with stirring to the former solution of hydroxyethylated starch and triphenylphosphine under ice cooling over 15 min. The resulting brown-colored solution was heated with stirring in an oil bath at 70°C for 18 hours. After completion of the reaction 10 ml of absolute methanol was added to the reaction mixture, which was then stirred at room temperature for 2 hours. The solvent was distilled off under reduced pressure while maintaining the reaction mixture at a temperature of 50°C or below to a concentrated volume of about 5-6 ml. To the residue was added 100 ml of chloroform, and the solution was extracted with 100 ml of distilled water. The aqueous layer was washed three times with 100 ml of chloroform and then passed through ion-exchange resin columns of 25 ml of Amberlite IR120B (H$^+$ type) and 25 ml of IRA 400 (OH$^-$ type), successively for deionization. The deionized solution was concentrated under reduced pressure at or below 40°C to about 10 ml. To the residual solution was added 60 ml of an acetone-isopropanol (1:1) solution, and a glutinous substance thus precipitated was isolated by decantation, to which was again added 60 ml of an acetone-isopropanol (1:1) solution to form powders. The powders were separated by filtration and dried. There was obtained 2.54 g of colorless powders.

m.p.: 194-199°C (dec.)

IR: $\nu \, ^{KBr}_{max}$ cm$^{-1}$

3380, 2930, 1625, 1360, 1010

Elementary analysis

Found %: C, 35.43; H, 5.78; I, 23.59

Limiting viscosity number: 0.05

Examples 4-18

A low viscosity hydroxyethylated starch was reacted with N-iodosuccinimide in the presence of triphenylphosphine in the same way as in Examples 1-3. There was obtained an iodized hydroxyethyl starch of low visocsity as desired.

Shown in Table 1 are molar ratios of N-iodosuccinimide (NIS) and triphenylphosphine (ø$_3$P) respectively to the starting low viscosity hydroxyethylated starch, reaction temperature, reaction time, yield (%), iodine content (%), limiting viscosity number and dissolution rate for the low viscosity hydroxyethylated starch used as the starting material in Examples 1-18. The solubility was measured by a method for the determination of relative dissolution rate, since measurement of the solubility when the concentration was high was difficult by the dissolution test method according to the pharmacopoeia of Japan. Relative dissolution rate was determined by measuring time required for entire dissolution of powders placed in distilled water and ultrasonicated. Ultrasonic-generating instrument used: Sharp Ultrasonic Cleaner, 150 W, 28 KHz; test tube: ø13 mm; concentration: 0.5 g/0.5 ml.

Table 1

| Example No. | Molar ratio to starting material | | Temperature (°C) | Time (hr) | Yield (%) | Iodine content (%) | Limiting viscosity number | Dissolution rate (min) |
|---|---|---|---|---|---|---|---|---|
| | NIS | ø₃P | | | | | | |
| 1 | 1.0 | 1.0 | 70 | 18 | 100.6 | 16.7 | 0.05 | 4.5 |
| 2 | 1.2 | 1.2 | 70 | 18 | 104.2 | 22.9 | 0.05 | 1.5 |
| 3 | 1.5 | 1.5 | 70 | 18 | 70.2 | 23.6 | 0.05 | 1.5 |
| 4 | 1.2 | 1.2 | Room temp. | 1 | 81.0 | 14.1 | 0.06 | |
| 5 | 1.2 | 1.2 | Room temp. | 41 | 84.1 | 13.9 | 0.05 | |
| 6* | 1.2 | 1.2 | Room temp. | 19.5 | 102.0 | 14.7 | | |
| 7 | 1.2 | 1.5 | Room temp. | 18 | 108.0 | 16.0 | 0.06 | |
| 8 | 1.2 | 1.2 | 40 | 18 | 61.0 | 16.3 | 0.06 | |
| 9** | 1.2 | 1.2 | 70 | 0 | 74.7 | 16.2 | 0.07 | |
| 10 | 1.2 | 1.2 | 70 | 1 | 65.0 | 13.9 | 0.06 | |
| 11 | 1.2 | 1.2 | 70 | 6 | 91.4 | 14.6 | 0.05 | |
| 12 | 1.2 | 1.2 | 70 | 12 | 98.7 | 21.4 | 0.05 | |
| 13 | 1.5 | 1.5 | Room temp. | 1 | 49.0 | 16.7 | 0.05 | |
| 14*** | 1.5 | 1.6 | Room temp. | 18 | 87.1 | 17.5 | | |
| 15* | 1.5 | 2.0 | Room temp. | 17 | 93.0 | 19.9 | | |
| 16 | 1.5 | 1.5 | Room temp. | 42 | 55.0 | 20.5 | 0.06 | |
| 17* | 1.5 | 1.5 | 40 | 17.5 | 108.2 | 20.0 | | |
| 18* | 1.5 | 1.7 | 70 | 1 | 86.1 | 20.5 | | |

Notes)

\* The solvent was used in an amount 5 times as much as in the other examples.

\*\* The treatment was initiated immediately after the NIS solution was added dropwise to the solution of the starting starch and ø₃P at 70° C over one min.

\*\*\* To the reaction solution was added, for HI trap, pyridine 0.5 times as many in moles.

Reaction using 1.7 moles or more of N-iodosuccinimide and triphenylphosphine respectively per mole of the hydroxyethyl group in the starting material and carried out at 70° C for 12 hours or longer afforded an iodized hydroxyethyl starch having an iodine content over 25%, which was insoluble in water.

Test Example

In order to evaluate the opacifying ability measurement was made of the CT number. In distilled water for injection were dissolved compounds of the invention and a control compound, respectively to prepare a 60% solution. In a phantom for measurement of relative absorption for CT solution (manufactured by Kyoto Kagaku Hyohon) was enclosed 10 ml of each of the solutions, and CT number was measured. The instrument used was a TCT60A-60 CT scanner, a scanning time of 3 sec. and a slice thickness of 10 mm being employed. The results are shown in Table 2. A compound described in an example of the above-cited Japanese Patent Publication No. 272201/1986 (6-iodoethylated starch) was used as a control.

Table 2

| Test material | CT number (HU) |
|---|---|
| Compound of Example 2 (Iodine content: 22.9%) | 1,906 |
| Compound of Example 3 (Iodine content: 23.6%) | 1,908 |
| Control (Iodine content: 12.5%) | 1,510 |

It is evident from the above table that the compounds of the present invention exert a superior opacifying ability.

## Claims

(1) An iodized hydroxyethyl starch of low viscosity characterized by having a limiting viscosity number of 0.03-0.13 and an iodine content of 13.5-25.0%.

(2) An iodized hydroxyethyl starch of low viscosity according to Claim 1 wherein the limiting viscosity number is 0.05-0.07 and the iodine content is 14-24%.

(3) A process for preparing an iodized hydroxyethyl starch of low viscosity according to Claim 1 which comprises reacting a hydroxyethylated starch having a limiting viscosity number of 0.04-0.18 with N-iodosuccinimide in the presence of triphenylphosphine.

(4) An X-ray contrast agent comprising an iodized hydroxyethyl starch of low viscosity according to Claim 1.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,Y | EP-A-0 206 551 (NIPPON UNIVERSAL PHARMACEUTICAL) * Abstract; page 4, lines 9-13; claim 2 * | 1-4 | C 08 B 31/12 A 61 K 49/04 |
| Y | US-A-3 937 821 (T. IRIKURA) * Abstract * | 1-4 | |
| A | DE-A-1 813 571 (AMERICAN HOSPITAL SUPPLY CORP.) * Claim 1 * | | |

TECHNICAL FIELDS SEARCHED (Int. Cl.5)

C 08 B
A 61 K

The present search report has been drawn up for all claims

| Place of search | Date of completion of the search | Examiner |
|---|---|---|
| THE HAGUE | 03-07-1990 | SOMERVILLE F.M. |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

 

& : member of the same patent family, corresponding
   document

EPO FORM 1503 03.82 (P0401)